# EUROPÄISCHE PATENTSCHRIFT

(11) **EP 1 474 110 B1**
(45) Veröffentlichungstag und Bekanntmachung des Hinweises auf die Patenterteilung: **08.08.2007**
(21) Anmeldenummer: 03739446.7
(22) Anmeldetag: 24.01.2003
(51) Int. Cl.: A61K 9/16, C08G 81/00, A61K 9/00

(54) **DEPOTARZNEIMITTEL MIT NEUEN KONJUGIERTEN TRÄGERN**
DEPOT MEDICAMENTS WITH NEW CONJUGATED CARRIERS
PRODUITS PHARMACEUTIQUES A LIBERATION PROGRESSIVE AVEC NOUVEAUX SUPPORTS CONJUGUES

(30) Priorität: 16.02.2002 DE 10206517
(43) Veröffentlichungstag der Anmeldung: 10.11.2004
(73) Patentinhaber: GELITA AG, 69412 Eberbach (DE)
(72) Erfinder: REICH, Gabriele, 69121 Heidelberg (DE); KÖHLER, Berthold, 69151 Neckargemünd (DE)
(74) Vertreter: Wössner, Gottfried
(86) Internationale Anmeldenummer: PCT/EP2003/000725
(87) Internationale Veröffentlichungsnummer: WO 2003/068192

(56) Entgegenhaltungen:
- EP-A- 0 467 389
- WO-A-96/28143
- DE-A- 19 813 174
- US-A- 6 034 175
- MORITA T ET AL: "Preparation of gelatin microparticles by co-lyophilization with poly(ethylene glycol): characterization and application to entrapment into biodegradable microspheres." INTERNATIONAL JOURNAL OF PHARMACEUTICS. NETHERLANDS 21 MAY 2001, Bd. 219, Nr. 1-2, 21. Mai 2001 (2001-05-21), Seiten 127-137, XP002244081 ISSN: 0378-5173
- LI J K ET AL: "A NOVEL BIODEGRADABLE SYSTEM BASED ON GELATIN NANOPARTICLES AND POLY(LACTIC-CO-GLYCOLIC ACID) MICROSPHERES FOR PROTEIN AND PEPTIDE DRUG DELIVERY" JOURNAL OF PHARMACEUTICAL SCIENCES, AMERICAN PHARMACEUTICAL ASSOCIATION. WASHINGTON, US, Bd. 86, Nr. 8, 1. August 1997 (1997-08-01), Seiten 891-895, XP000693967 ISSN: 0022-3549 in der Anmeldung erwähnt
- NAM Y S ET AL: "PROTEIN LOADED BIODEGRADABLE MICROSPHERES BASED ON PLGA-PROTEIN BIOCONJUGATES" JOURNAL OF MICROENCAPSULATION, TAYLOR AND FRANCIS INC. LONDON, GB, Bd. 16, Nr. 5, September 1999 (1999-09), Seiten 625-637, XP000834854 ISSN: 0265-2048 in der Anmeldung erwähnt
- WYBOR W ET AL: "BIODEGRADABLE POLYMERS. 1. GRAFTING OF VINYL POLYMERS ON TO GELATIN" PROGRESS IN RUBBER AND PLASTICS TECHNOLOGY, PLASTICS AND RUBBER INSTITUTE. LONDON, GB, Bd. 16, Nr. 2, 1. April 2000 (2000-04-01), Seiten 117-133, XP000950136 ISSN: 0266-7320

## Beschreibung

Die Erfindung betrifft ein Depotarzneimittel mit einem pharmakologischen Wirkstoff und einem Träger, insbesondere Depotarzneimittel zur parenteralen Verabreichung, sowie Trägermaterialien für Depotarzneimittel und ein Verfahren zur Herstellung dieser Trägermaterialien.

Depotarzneimittel, insbesondere solche, die parenteral oder auch oral verabreicht werden können, gewinnen stetig an Bedeutung, da sie nicht nur eine kontrollierte Freigabe der im Träger eingeschlossenen Wirkstoffe über einen längeren Zeitraum erlauben und damit einen gleichmäßigen Blutspiegel des Wirkstoffs im Körper, sondern sie erlauben darüber hinaus die gezielte Anwendung der Wirkstoffe und geben instabilen Wirkstoffen einen Schutz.

Als Trägermaterialien wurden schon eine Vielzahl verschiedener biologisch abbaubarer Polymere eingesetzt, insbesondere Polyester, wie z.B. Polylactide und Polypeptide, wobei alle Systeme mit erheblichen Nachteilen behaftet sind.

So beschreiben R. Mank et al. in Pharmazie 46 (1991), Seite 9 bis 18, "Parenterale Depotarzneiformen auf der Basis von biologisch abbaubaren Polymeren", verschiedene, prinzipiell mögliche Ausgangsmaterialien zur Herstellung von Trägermaterialien für Depotarzneimittel, nämlich Polyester und Polypeptide.
In der Folgezeit haben dann verschiedene Studien, z.B. L. Meinel et al., Journal of Controlled Release 70 (2001), Seiten 193 bis 202, "Stabilizing insuline-like growth factor-I in poly(D,L-lactide-co-glycolide) microspheres", die Verwendung von Polyestern als Trägermaterial für pharmazeutische Wirkstoffe beschrieben.

Ebenfalls auf der Basis von Poly(D,L-lactid-co-glycoliden) arbeiten Y. S. Nam und T. G. Park, J. Microencapsulation 16 (1999), Seiten 625 bis 637, "Protein loaded biodegradable microspheres based on PLGA-protein bioconjugates", wobei dort an das Polylactid ein Wirkstoffprototyp (Lysozym) chemisch gekoppelt wird.

Mit Gelatine-PLGA-Mischungen arbeiten J. K. Li et al., Journal of Pharmaceutical Sciences 86 (1997), Seiten 891 bis 895, "A Novel Biodegradable System Based on Gelatin Nanoparticles and Poly(lactid-*co*-glycolic acid) Microspheres for Protein and Peptide Drug Delivery". Eine weitere Druckschrift, nämlich WO 94/15587, verwendet ionische Molekularkonjugate von bioabbaubaren Polyestern und biologisch aktiven Polypeptiden.

Als weiteres Beispiel sei schließlich noch die Veröffentlichung von A. Kosasih et al. in International Journal of Pharmaceutics 204 (2000), Seiten 81 bis 89, "Characterization and in vitro release of methotrexate from gelatin/methotrexate conjugates formed using different preparation variables", erwähnt, wo Gelatine/Metothrexat-Konjugate zur Depotarzneimittelherstellung verwendet werden.

Polypeptide sind an sich ein geeignetes Material als bioabbaubare Träger, jedoch ist ihre Auflösegeschwindigkeit in der Regel deutlich zu groß, so dass sie nach parenteraler Applikation nicht ausreichend vor proteolytischem Abbau schützen bzw. bei oraler Applikation eine fast momentane Freisetzung der Wirkstoffe einhergeht. Insbesondere Peptidwirkstoffe lassen sich über Polypeptidträgermaterialien nicht sicher durch den Magen-Darm-Trakt bringen, da die saure Umgebung im Magen sowohl zu einer schnellen hydrolytischen Zersetzung der Trägermaterialien als auch des Wirkstoffes führt.

Demzufolge wurden in der Literatur unlösliche Polymere, z.B. Polylactide, als Polyester bevorzugt, die im wässrigen Milieu wesentlich langsamer abbauen.

Beim biologischen Abbau von Polylactiden entstehen allerdings Protonen in wässriger Lösung, die die Stabilität der pharmakologischen Wirkstoffe beeinträchtigen können und insbesondere zum Abbau derselben oder zu deren Denaturierung führen können.

Bei reinen Polylactidpartikeln findet eine beschleunigte Zersetzung des Polymermaterials zunächst hauptsächlich im Inneren statt (heterogener Bulkabbau), da sich dort ein saures Medium bildet, das sich mit der Umgebung nicht austauscht, so dass die sich in der Folge im Partikelinneren bildende höhere Wasserstoffionenkonzentration quasi autokatalytisch zu einem beschleunigten weiteren Abbau des Polylactidpartikelinneren führt. Im Partikelinneren sind jedoch in der Regel die größten Anteile des Wirkstoffs vorhanden, so dass, bis ein Austausch der flüssigen Phase im Inneren des Partikels mit der Umgebung stattfindet, der größte Teil des im Partikel enthaltenen Wirkstoffs durch die Wasserstoffionenkonzentration modifiziert oder auch bereits denaturiert wurde. Daraus resultiert eine unregelmäßige, im Vorhinein nicht bestimmbare Wirkstofffreisetzung.

Aufgabe der vorliegenden Erfindung ist es, ein Depotarzneimittel der eingangs beschriebenen Art vorzuschlagen, bei dem eine definierte Wirkstoffabgabe über die Zeit erhalten wird und bei dem der Wirkstoff in seiner pharmakologisch wirksamen Form erhalten bleibt.

Diese Aufgabe wird bei dem eingangs beschriebenen Depotarzneimittel erfindungsgemäß dadurch gelöst, dass der Träger unter Verwendung eines Trägermaterials hergestellt ist, welches ein aus einem Polypeptid und einem hiermit kovalent verknüpften, biologisch abbaubaren Polyester gebildetes Trägerpolymer umfasst.

Erstaunlicherweise lässt sich durch die Verwendung eines solchen neuartigen Trägerpolymers zum einen erreichen, dass die Wirkstoffabgabe über die Zeit definiert ist, d.h. gegenüber der Wirkstofffreisetzung von in Polypeptiden inkorporierten Wirkstoffen über einen deutlich längeren Zeitraum verteilt ist, und darüber hinaus eine Denaturierung des Wirkstoffes selbst im wesentlichen vermieden wird.

Bei dem erfindungsgemäßen Trägerpolymer erfolgen Quellung, Abbau und Auflösung homogen. Dies ermöglicht zum einen ein kontinuierliches Herausdiffundieren des Wirkstoffes aus den Trägerpolymerpartikeln und bedeutet weiterhin, dass sich im Inneren des Partikels kein saures Milieu bilden kann, da dieses Innere des Partikels mit der äußeren wässrigen Umgebung in Verbindung steht und gleichzeitig der Polypeptidanteil als Puffersubstanz wirkt.

Das Trägerpolymer kann dazu verwendet werden, eine Wirkstoffphase zu umschließen, so dass der Wirkstoff quasi verkapselt oder in einer Matrix vorliegend zum Einsatz kommt. Ferner ist es vorstellbar, dass das Trägerpolymer den pharmakologischen Wirkstoff durch Adsorption aufnimmt und/oder in vorhandenen Poren eingelagert enthält.

Bevorzugt beträgt der Anteil des chemisch gebundenen Polyesters in dem Trägerpolymer 1 Mol-% oder mehr. Das bedeutet, dass beim Umsetzen von Polyester mit Polypeptid mindestens 1 Mol-% des eingesetzten Polyesters mit Polypeptid chemisch verknüpft vorliegt. Ungebundene Anteile des Polyesters und/oder des Polypeptids können in der Mischung verbleiben oder bei speziellen Anforderungen durch einen Aufarbeitungsprozess abgetrennt werden. Über den Anteil des Polyesters an dem Trägerpolymer lässt sich Einfluss nehmen auf die Depotwirkung der Arzneimittel bzw. auf den vorgesehenen Zeitraum für die Freisetzung des Wirkstoffes.

Das Gewichtsverhältnis von Polyester zu Polypeptid in dem Trägerpolymeren variiert bevorzugt im Bereich von 1:99 bis 99:1, bevorzugt im Bereich von 30:70 bis 99:1. Über die Variation dieses Verhältnisses lässt sich die Abbaurate des Trägerpolymeren in weiten Grenzen variieren und korrespondierend hierzu die Wirkstofffreisetzung.

Bei jedem der Grenzwerte findet man eine merkliche Veränderung des Abbauverhaltens des Trägerpolymeren gegenüber den jeweiligen Ausgangspolymeren. Insbesondere macht sich bereits 1 Gew.% Polypeptid mit seiner Pufferwirkung beim Abbauprozess des Trägerpolymeren bemerkbar.

Erfahrungsgemäß sind bei bevorzugten Trägerpolymeren für Depotarzneimittel bereits Polypeptidanteile von 2 Gew.%, insbesondere 3 Gew.%, genügend, um eine optimale Wirkung für die Freisetzung und auch den Schutz des pharmakologischen Wirkstoffes durch den Träger zu gewährleisten. Am meisten bevorzugt sind Trägerpolymere mit 10 Gew.% Polypeptid oder auch mehr.

Bei den Polyestern empfehlen sich insbesondere Polyglykolide, Poly(D,L-lactid-coglycolide), Polyalkylenglykol-Polylactid-Blockcopolymere, Polyalkylenglykol-PLGA-Blockcopolymere, POE-POP-PLA-Blockcopolymere, POE-PLGA-Blockcopolymere, Poly-ε-caprolactame, stereoisomere Lactid-Oligomere, Oligolactide (n ≥ 3) oder Polylactide.

Das Polypeptid des Trägerpolymers wird vorzugsweise ausgewählt aus Kollagen, Gelatine, globulären Proteinen und Albuminen oder anderen Hydrogele bildenden Proteinen, Enzymen und Abbauprodukten von Proteinen.

Als Basis für die Polypeptide kommen sowohl Säugetiere-Kollagen-Materialien und deren Abbauprodukte, insbesondere Gelatine, zum Einsatz, wie z.B. Rindergelatine, Schweinegelatine, Schafgelatine, aber auch Geflügel- und Fischgelatine. Selbstverständlich eignen sich auch gentechnisch hergestellte Proteinmaterialien wie z.B. Gelatine, Kollagen oder Kollagenfragmente, insbesondere auch solche, die auf Pflanzenbasis hergestellt sind, und die im Hinblick auf die derzeit geführte BSE-Diskussion künftig an Bedeutung gewinnen können. Ebenso eignen sich chemisch und/oder enzymatisch modifizierte Polypeptide.

Die Gelatine kann einen Bloomwert im Bereich von 30 - 320 aufweisen oder hydrolysiert sein und nach dem sauren Verfahren (Typ A) oder dem alkalischen Verfahren (Typ B) oder durch Druck/Temperatur oder auf enzymatischem Wege gewonnen sein.

Als weiterer Bestandteil der Trägerpolymere eignen sich herkömmliche Weichmacher, Puffersubstanzen, Tenside, Lipide und Porenbildner.

Bei den besonders bevorzugten Trägerpolymeren wird die Verknüpfung von Polypeptid und Polyester über eine freie Hydroxylfunktion des Polyesters mit einer reaktiven Gruppe des Polypeptids erfolgen.

Alternativ, aber auch ergänzend ist es möglich, dass die Verknüpfung von Polypeptid und Polyester über eine freie Carboxylfunktion des Polyesters oder nach erfolgter Oxidation einer Hydroxylfunktion zu einer Carbonylgruppe über diese mit einer reaktiven Gruppe des Polypeptids erfolgt.

Die bevorzugte reaktive Gruppe des Polypeptids ist die Aminofunktion.

Im Hinblick auf den bevorzugten Weg der Verknüpfung sind lysinhaltige Polypeptide zu bevorzugen, wobei ein Anteil von beispielsweise 3 % an Lysingruppen, gegebenenfalls auch modifizierten Lysingruppen, sehr gute Ergebnisse bringt.

Die Erfindung betrifft ferner ein Verfahren zur Herstellung eines Trägermaterials für Depotarzneimittel, welches dadurch gekennzeichnet ist, dass ein Polyester in einem ersten Schritt mit aktivierten Hydroxylgruppen hergestellt und in einem zweiten Schritt ein Polypeptid zugegeben und mit seinen reaktiven Gruppen mit den aktivierten Hydroxylgruppen des Polyesters unter Ausbildung einer kovalenten Bindung umgesetzt wird.

Hierbei entsteht ein neues Block-Copolymer, das sich ausgezeichnet als Trägermaterial für Depotarzneimittel eignet.

Die Aktivierung der Hydroxylgruppe wird so durchgeführt, dass diese in eine gute Austrittsgruppe überführt wird.

Als Polyester eignet sich ein sehr breiter Bereich an Verbindungen mit unterschiedlichen Molekulargewichten (beispielsweise 2.000 bis 300.000), insbesondere auch die verschiedensten Poly(D,L-lactide) oder Poly(D,L-lactid-co-glycolide).

Bei den zuletzt genannten Polyestern lassen sich die unterschiedlichsten Verhältnisse von Glykolsäure sowie D- oder L-Milchsäure verwenden. Ebenso verwendbar sind Polyester mit modifizierten Esterendgruppen, Polyalkylenglykole, Polypropylenoxid-Polyethylenoxid-Polylactid-Coglykolid-Blockcopolymere (POP-POE-PLGA-Blockcopolymere), und verschiedene Kombinationen dieser Polymere sind ebenfalls als Ausgangsmaterial geeignet.

Geeignete Reagentien, um die Aktivierung der Hydroxylgruppe des Polyesters herbeizuführen, sind beispielsweise p-Toluol-sulfonylchlorid, Methansulfonylchlorid, p-Bromobenzolsulfonylchlorid, p-Nitrobenzolsulfonylchlorid, Trifluormethansulfonylchlorid und andere, die dem Fachmann für die Bildung von guten Austrittsgruppen her bekannt sind.

Da die meisten Reagentien wasserempfindlich sind, muss diese Reaktion in einem trockenen, organischen Lösemittel am besten unter Stickstoffatmosphäre durchgeführt werden.

Als Lösemittel eignen sich Tetrahydrofuran, Dichlormethan, Chloroform und andere.

Um die bei der Reaktion freigesetzten Wasserstoffionen abzufangen, lassen sich verschiedene Basen, wie z.B. Diethylamin, Triethylamin, Hünig-Base, Pyridin, 2,6- Di(tert-butyl)-4-methylpyridin und andere der Reaktionsmischung zusetzen. Das sich ergebende Produkt wird in herkömmlicher Weise isoliert. Die einzusetzenden Polypeptide sind die bereits oben erwähnten und insbesondere solche, die Hydrogele formen, wie z.B. Gelatine.

Um die Reaktivität des aktivierten Polyesters einzustellen, kann die aktivierte Gruppe auch in eine Halogenidverbindung umgewandelt werden. Hierzu empfiehlt sich eine modifizierte Finckelstein-Reaktion, wobei die korrespondierenden Jodid-oder Bromidverbindungen erhalten werden können.

Halogenidverbindungen können außerdem direkt aus der Hydroxylgruppe erhalten werden, unter Verwendung von beispielsweise Thionylchlorid, das mehr sensitive Triphenylphosphindibromid oder Triphenylphosphin in CCl₄.

Der aktivierte Polyester kann nun mit jeder nucleophilen Gruppe des Polypeptids, wie z.B. Aminogruppen oder Hydroxylgruppen, zur Reaktion gebracht werden. Diese Reaktion wird in einem polaren Lösemittel (z.B. DMF oder DMSO) oder in Lösemittelmischungen wie z.B. Ethylacetat/Wasser, Aceton/Wasser, THF/Wasser, CHCl₃/Wasser und CH₂Cl₂/Wasser durchgeführt.

Die Temperatur der Reaktion und die Reaktionszeit können variiert werden, je nach Reaktivität der verwendeten Reagentien.

Nach der Beendigung der Reaktion wird das Lösemittel abgedampft, und das Produkt wird weiter getrocknet, wobei ein weißes oder leicht gelbliches Pulver erhalten wird.

Das Vorhandensein einer neuen Verbindung wurde mittels IR- und NIR-Spektroskopie und Differential Scanning Calorimetry (DSC) nachgewiesen, wobei ein neuer Glaspunkt für das Produkt beobachtet werden kann. Darüber hinaus ist die Löslichkeit des Produktes vollständig verschieden von den Ausgangsmaterialien.

Diese und weitere Vorteile der Erfindung werden im Folgenden an Hand der Beispiele noch näher erläutert.

### Beispiele

### Beispiel 1:

### Aktivierung der Hydroxylgruppen der Polyesterkomponente

In einem trockenen Reaktionskolben mit Inhalt 50 ml werden unter einer Stickstoffatmosphäre 3,4 g Resomer RG503H (Poly(D,L-lactid-co-glycolid) mit einem Molekulargewicht von 34.000, Glaspunkt 49,9° C der Fa. Boehringer Ingelheim) in 10 ml trockenem Dichlormethan aufgelöst. Andere, ähnlich gut einsetzbare Lösemittel sind THF, CHCI₃ und Ethylacetat. Dazu werden 104 mg Triethylamin (über KOH getrocknet) zugegeben. Die Mischung wird für 15 min auf 0 °C gekühlt, dann werden 114 mg Methansulfonylchlorid über eine Spritze zudosiert. Die Reaktionsmischung wird während einer Stunde bei Raumtemperatur gerührt und dann zur Hydrolyse in eine Eis/Wasser-Mischung gegossen. Die wässrige Phase wird dreimal mit Dichlormethan extrahiert und die vereinigten organischen Phasen werden mit einer gesättigten Natriumbicarbonatlösung gewaschen. Nach der Extraktion wird die organische Phase über Magnesiumsulfat getrocknet. Das Lösemittel wird abgedampft und eventuell verbleibende Lösemittelrückstände werden in einem hohen Vakuum entfernt.

### Koppeln der Polyesterkomponente mit einem Polypeptid

Die frisch zubereitete aktivierte Polyesterkomponente wird in 20 ml Chloroform gelöst und zu 20 ml einer wässrigen Lösung von 3,7 g Schweinehautgelatine (hochbloomig Typ A, Glaspunkt 67,9° C) zugegeben. Die Mischung wird während 6 Stunden bei einer Temperatur von 55 °C (Ölbadtemperatur) gerührt und dann über Nacht bei Raumtemperatur gerührt. Das Lösemittel wird verdampft, soweit dies in einem Rotationsverdampfer möglich ist. Das verbleibende Wasser wird in einem Exikator, der ein Trockenmittel enthält, entfernt.

Nicht umgesetztes PLGA wird durch Extraktion mit Methylenchlorid entfernt. Der unlösliche Rückstand wird im Vakuum getrocknet und anschließend in DMSO aufgenommen. Das erfindungsgemäße Produkt geht dabei in Lösung. Anschließend wird die das erfindungsgemäße Produkt enthaltende DMSO-Lösung sprühgetrocknet. Je nach Sprühbedingungen erhält man sphärische Partikel im Bereich von 1-10 µm Durchmesser.

Das erfindungsgemäße Produkt weist ein Verhältnis Polyester : Polypeptid von 90: 10 und einen Glaspunkt von 55,9° C auf.

Zur Bestimmung der Glaspunkte wurden alle untersuchten Materialien zuvor bei 25° C und 25 % relativer Feuchte konditioniert.

Die IR- und NIR-Daten des erfindungsgemäßen Produkts unterscheiden sich signifikant sowohl von den Ausgangsverbindungen als auch von überlagerten Spektren der Ausgangssubstanzen.

Im Gegensatz zur Ausgangsgelatine ist das erfindungsgemäße Produkt vollständig in DMSO löslich. In sonst üblichen Lösemitteln wie Alkohlen, Aceton (Ketone), Ethern und Wasser ist das Produkt unlöslich.

Durch Auswahl des Polyestertyps, dessen Kettenlänge und dem Verhältnis von Gelatine zu Polyesteranteil in dem erfindungsgemäßen Produkt sowie der Art der Endgruppen des Polyesters läßt sich die Abbaukinetik in weiten Grenzen variieren.

In gewissen Grenzen läßt sich die Abbaukinetik des erfindungsgemäßen Produkts auch durch entsprechende Auswahl der Gelatine beeinflussen.

### Beispiel 2:

In derselben Weise wie in Beispiel 1 werden verschiedene Polyesterkomponenten umgesetzt, nämlich PLGA, PLA und POP-POE-PLGA-Copolymere mit unterschiedlichen Verhältnissen der zwei Polymergruppen. Die Umsetzung war in jedem der Fälle erfolgreich, und zwar unabhängig vom Hersteller der jeweiligen Polyesterkomponenten (getestet wurden Verbindungen der Firmen Boehringer Ingelheim, Medisorp und Vako).
Die Ergebnisse bezüglich Ausbeute an erfindungsgemäßem Produkt sind vergleichbar mit denen von Beispiel 1.

### Beispiel 3:

Zur Aktivierung der Hydroxylfunktion der Polyesterkomponente wurde p-Toluol-sulfonylchlorid verwendet, wobei sich gegenüber der Vorgehensweise in Beispiel 1 eine vereinfachte Verfahrensweise ergibt:

In einem Reaktionsgefäß mit Inhalt 50 ml werden 3,4 Resomer RG503H in 10 ml destilliertem Dichlormethan aufgelöst. Dazu werden 104 mg Triethylamin (p.a.) zugegeben. Die Mischung wird für 15 min. auf 0 °C abgekühlt, und 190 mg p-Toluol-sulfonylchlorid werden zugegeben. Die Reaktionsmischung wird während einer Stunde bei Raumtemperatur gerührt und dann zur Hydrolyse in eine Eis/Wasser-Mischung gegossen. Die wässrige Phase wird dreimal mit Dichlormethan extrahiert, und die zusammengeführten organischen Phasen der Extraktionsschritte werden mit einer gesättigten wässrigen Natriumbicarbonatlösung gewaschen. Nach dem Waschen mit der Natriumbicarbonatlösung wird die organische Phase über Magnesiumsulfat getrocknet, das Lösemittel abgedampft und verbleibende Lösemittelreste in einem hohen Vakuum entfernt.

Der Vorteil dieser Vorgehensweise liegt darin, dass nicht unter Wasserausschluss gearbeitet werden muss.

Die frisch hergestellte aktivierte Polyesterkomponente wird in 20 ml Chloroform aufgelöst, und hierzu werden 20 ml einer wässrigen Lösung von 3,7 g Schweinehautgelatine (Typ A) zugegeben. Die Mischung wird während sechs Stunden bei einer Temperatur von 55 °C (Ölbadtemperatur) gerührt und dann über Nacht bei Raumtemperatur gerührt. Das Lösemittel wird verdampft, soweit dies mit einem Rotationsverdampfer möglich ist. Verbleibendes Wasser wird in einem Exikator mit einem Trockenmittel entfernt. Die Eigenschaften des erhaltenen Produkts entsprechen denen von Beispiel 1.

### Beispiel 4:

In einem trockenen Kolben mit Inhalt 50 ml werden unter Stickstoffatmosphäre 3,4 g Resomer RG503H in 10 ml trockenem Dichlormethan gelöst. 104 mg Triethylamin (getrocknet über KOH) werden hinzugegeben. Die Mischung wird für 15 min. bei 0 °C gekühlt, und mit einer Spritze werden 114 mg Methansulfonylchlorid zugegeben. Die Reaktionsmischung wird eine Stunde lang bei Raumtemperatur gerührt und dann zur Hydrolyse in eine Eis/Wasser-Mischung eingegossen. Die wässrige Phase wird dreimal mit Dichlormethan extrahiert, und die zusammengegebenen organischen Phasen werden mit gesättigter Natriumdicarbonatlösung gewaschen. Nach dem Waschen der organischen Phase wird diese über Magnesiumsulfat getrocknet. Das Lösemittel wird verdampft, und die verleibenden Lösemittelreste werden in hohem Vakuum entfernt.

Abweichend von der in Beispiel 1 beschriebenen Vorgehensweise wird hier in einer modifizierten Finckelstein-Reaktion die Polyesterkomponente mit aktivierten Hydroxylgruppen (die hier in Sulfonatgruppen umgewandelt sind) in ein Jodid oder Bromid umgewandelt, in dem einfach die Verbindung mit einem Überschuss an Natriumjodid bzw. Lithiumbromid in trockenem Aceton als Lösemittel gerührt wird. Nachdem die Mischung einen Tag lang (bei Raumtemperatur) gerührt wurde, wird diese abgefiltert und das Aceton von dem resultierenden Produkt entfernt.

Über die nachgelagerte Finckelstein-Reaktion lässt sich die Reaktivität und damit der Umsetzungsgrad der Kopplungsreaktion beeinflussen.

### Kopplungsreaktion der Polyesterkomponente mit der Polypegtidkomponente

Die frisch zubereitete Polyesterkomponente (siehe oben) wird in 20 ml Chloroform gelöst, und hierzu werden 20 ml einer wässrigen Lösung von 3,7 g Schweinehautgelatine (Typ A) zugegeben. Alternative Lösemittel sind hier beispielsweise Wasser/Dichlormethan 1:1, Wasser/Ethylacetat 1:1, DMF und DMSO. Die Mischung wird während sechs Stunden bei 55 °C (Ölbadtemperatur) gerührt und dann über Nacht bei Raumtemperatur gerührt. Das Lösemittel wird verdampft, soweit dies mit einem Rotationsverdampfer möglich ist. Verbleibendes Wasser wird in einem Exikator, der ein Trockenmittel enthält, entfernt.

### Beispiel 5:

Dieselbe Vorgehensweise wie in Beispiel 1 wird angewendet, jedoch wird hier an Stelle der Schweinehautgelatine vom Typ A eine Typ B-Gelatine verwendet, welche aus Knochen oder Rinderhaut hergestellt ist. Die Kopplungsreaktion von Polypeptidkomponente und Polyesterkomponente konnte in gleicher Weise durchgeführt werden wie zuvor beschrieben.

### Beispiel 6:

Dieselbe Herstellungsmethode wie in Beispiel 1 wird angewendet, jedoch wird enzymatisch hydrolysierte Gelatine (Gelita-Collagel^{®} A) bei der Kopplungsreaktion der Polypeptidkomponente mit der aktivierten Polyesterkomponente verwendet.

### Beispiel 7:

Dieselbe Präparationsmethode wie in Beispiel 1 wird angewendet, jedoch wird Gelatinehydrolysat (enzymatisch hydrolysierte Gelatine wie z.B. Gelita-Sol^{®} D/Sol DA und andere) für die Kopplungsreaktion des Polypeptids mit dem aktivierten Polyester verwendet.

### Beispiel 8:

Dieselbe Herstellungsmethode wie in Beispiel 1 wird angewendet, jedoch wird Ovalbumin als Polypeptidkomponente für die Kopplungsreaktion mit dem aktivierten Polyester verwendet.

### Beispiel 9:

Dieselbe Präparationsmethode wie in Beispiel 1 wird angewendet, jedoch wird die Kopplungsreaktion der aktivierten Polyesterkomponente mit dem Polypeptid in basischer Lösung durchgeführt. Die Basizität des Reaktionsmediums bewirkt eine Reaktivitätssteigerung und somit eine Verkürzung der Reaktionszeit.

Dieselbe Präparationsmethode wie in Beispiel 1 kann angewendet werden, wobei jedoch die Kopplungsreaktion der aktivierten Polyesterkomponente mit der Polypeptidkomponente in einer sauren Lösung durchgeführt wird. Die Reaktionszeit wird durch das saure Milieu des Reaktionsmediums vermindert.

### Beispiel 10:

Dieselbe Herstellungsmethode wie in Beispiel 1 wird angewendet, jedoch wird die Kopplungsreaktion der aktivierten Polyesterkomponente mit der Polypeptidkomponente bei Raumtemperatur durchgeführt bei gegenüber Beispiel 4 verlängerter Reaktionszeit.

Dieselbe Präparationsmethode wie in Beispiel 1 kann angewendet werden, wobei jedoch die Reaktion der aktivierten Polyesterkomponente mit der Polypeptidkomponente unter Rückfluss der Reaktionsmischung durchgeführt wird bei entsprechend verminderter Reaktionszeit.

### Beispiel 11:

An Hand dieses Beispiels soll das Einbringen eines Wirkstoffes in das Trägermaterial und somit die Herstellung eines Depotarzneimittels beschrieben werden, wobei als Modellwirkstoff das Enzym Lysozym verwendet wird. Es wird sowohl die Freisetzungskinetik als auch die Aktivität des freigegebenen Modellwirkstoffs bestimmt.

Der Modellwirkstoff Lysozym wird in einer Menge von 10 Gew.% bezogen auf den Gesamtfeststoffgehalt, der bei der Aufarbeitung des Reaktionsproduktes der Kopplungsreaktion von Beispiel 1 enthaltenen DMSO-Lösung in diese eingebracht wird, und die Lösung wird dann in gleicher Weise wie in Beispiel 1 beschrieben sprühgetrocknet. Die dabei erhaltenen Partikel weisen dem entsprechend einen Lysozymgehalt von 10 Gew.% auf.

Das Abbauverhalten des erfindungsgemäßen Trägerpolymeren und die damit korrespondierende Freisetzung des Modellwirkstoffes Lysozym wurde in einer isotonischen PBS-Lösung mit Serumhydrolasen bei einer Temperatur von 37 °C und einem pH-Wert von 7,4 ermittelt.

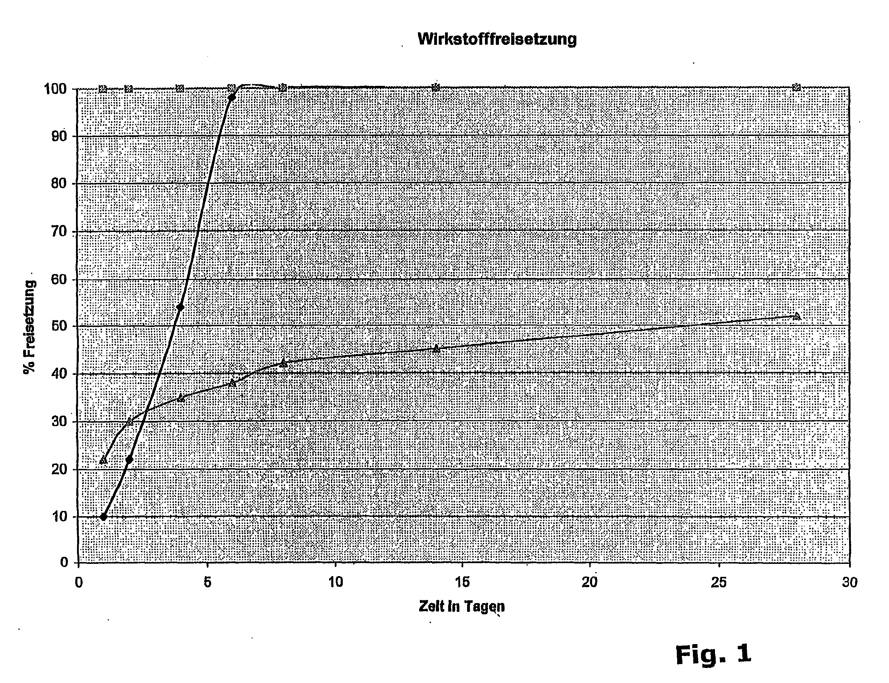

In Figur 1 sind drei Freisetzungskurven des Modellwirkstoffs Lysozym aus Mikropartikeln, bestehend aus Polypeptid (■) (hochbloomige Gelatine Typ A, Glaspunkt 67,9 °C), Polyester (▲) (Resomer RG503H) und dem erfindungsgemäßen Trägerpolymeren (◆) im Verlauf von 28 Tagen gezeigt. Den drei Polymertypen waren, wie eingangs dieses Beispiels beschrieben, 10 Gew.% Lysozym als Modellwirkstoff zugesetzt worden.

Das Polypeptid (Gelatine) zeigt eine im gewählten Zeitmaßstab praktisch sofortige 100%ige Freisetzung des Modellwirkstoffes Lysozym.

Die Freisetzung von Lysozym aus dem Polyester ist selbst nach 28 Tagen noch nicht abgeschlossen. Mit zunehmender Inkubationszeit beobachtet man eine fortschreitende Inaktivierung des Wirkstoffs und eine allmähliche Stagnation der Freigabe.

Die Freisetzung aus dem erfindungsgemäßen Trägerpolymeren des Beispiels 1 erfolgt sehr gleichmäßig über fünf Tage hinweg ohne Beeinträchtigung des Aktivität des Wirkstoffs. Vergleichbare Freisetzungsverläufe erhält man mit den gemäß den Beispielen 2 bis 10 hergestellten erfindungsgemäßen Trägermaterialien.

## Patentansprüche

1. Depotarzneimittel mit einem pharmakologischen Wirkstoff und einem Träger, insbesondere für die parenterale Applikation, **dadurch gekennzeichnet, dass** der Träger unter Verwendung eines Trägermaterials hergestellt ist, welches ein aus einem Polypeptid und einen hiermit kovalent verknüpften, biologisch abbaubaren Polyester gebildetes Trägerpolymer umfasst.

2. Depotarzneimittel nach Anspruch 1, **dadurch gekennzeichnet, dass** das Gewichtsverhältnis von Polyesteranteil zu Polypeptidanteil von 1:99 bis 99:1 beträgt.

3. Depotarzneimittel nach Anspruch 1 oder 2, **dadurch gekennzeichnet, dass** das Gewichtsverhältnis von Polyesteranteil zu Polypeptidanteil 30:70 bis 99:1 beträgt.

4. Depotarzneimittel nach einem der Ansprüche 1 bis 3, **dadurch gekennzeichnet, dass** der Polyester ausgewählt ist aus Polyglykoliden, Poly(D,L-lactid-co-glycoliden), Polyalkylenglykol-Polylactid-Blockcopolymeren, Polyalkylenglykol-PLGA-Blockcopolymeren, POP-POE-PLA-Blockcopolymeren, POP-PLA-Blockcopolymeren, POP-PLGA-Blockcopolymeren, POE-PLGA-Blockcopolymeren, Poly-ε-caprolactamen, stereoisomeren Lactid-Oligomeren, Oligolactide (n ≥ 3) oder Polylactiden.

5. Depotarzneimittel nach einem der Ansprüche 1 bis 4 , **dadurch gekennzeichnet, dass** das Polypeptid ausgewählt ist aus Kollagen, Gelatine, globulären Proteinen oder anderen Hydrogele bildenden Proteinen, Enzymen und Abbauprodukten von Proteinen, welche chemisch und/oder enzymatisch modifiziert sein können.

6. Depotarzneimittel nach einem der Ansprüche 1 bis 5, **dadurch gekennzeichnet, dass** die Verknüpfung von Polypeptid und Polyester über eine freie Hydroxyfunktion des Polyesters mit einer reaktiven Gruppe des Polypeptids erfolgt ist.

7. Depotarzneimittel nach einem der Ansprüche 1 bis 6, **dadurch gekennzeichnet, dass** die Verknüpfung von Polypeptid und Polyester über eine freie Carboxylfunktion des Polyesters oder eine durch Oxidation der Hydroxylfunktion erhaltene Carbonylfunktion mit einer reaktiven Gruppe des Polypeptids erfolgt ist.

8. Depotarzneimittel nach Anspruch 6 oder 7, **dadurch gekennzeichnet, dass** die reaktive Gruppe des Polypeptids eine Aminofunktion ist.

9. Verfahren zur Herstellung eines Trägermaterials für Depotarzneimittel, **gekennzeichnet durch** die Schritte
Umsetzen einer Polyesterkomponente mit einem aktivierenden Agens, um Hydroxylgruppen der Polyesterkomponente in eine aktivierte Form zu überführen,
Zugeben einer Polypeptidkomponente und Reagierenlassen von reaktiven Gruppen des Polypeptids mit den aktivierten Hydroxylgruppen des Polyesters unter Ausbildung von kovalenten Bindungen.

10. Verfahren nach Anspruch 9, **dadurch gekennzeichnet, dass** die Hydroxylgruppen mit dem aktivierenden Agens in gute Austrittsgruppen umgewandelt werden.

11. Verfahren nach Anspruch 9 oder 10, **dadurch gekennzeichnet, dass** die Polyesterkomponente ausgewählt ist aus Polyglykoliden, Poly(D,L-lactid-co-glycoliden), Polyalkylenglykol-Polylactid-Blockcopolymeren, Polyalkylenglykol-PLGA-Blockcopolymeren, POP-POE-PLA-Blockcopolymeren, POP-PLA-Blockcopolymeren, POP-PLGA-Blockcopolymeren, POE-PLGA-Blockcopolymeren, Poly-ε-caprolactamen, stereoisomeren Lactid-Oligomeren, Oligolactide (n ≥ 3) oder Polylactiden.

12. Verfahren nach einem der Ansprüche 9 bis 11, **dadurch gekennzeichnet, dass** die Polypeptidkomponente ausgewählt ist aus Kollagen, Gelatine, globulären Proteinen oder anderen Hydrogele bildenden Proteinen, Enzymen und Abbauprodukten von Proteinen.

13. Verfahren nach einem der Ansprüche 9 bis 12, **dadurch gekennzeichnet, dass** das aktivierende Agens ausgewählt ist aus p-Toluol-sulfonylchlorid, Methansulfonylchlorid, p-Bromobenzolsulfonylchlorid, p-Nitrobenzolsulfonylchlorid und Trifluoromethansulfonylchlorid.

14. Verfahren nach einem der Ansprüche 9 bis 13, **dadurch gekennzeichnet, dass** der Schritt der Aktivierung der Hydroxylgruppen der Polyesterkomponente in einem Lösemittel durchgeführt ist, welches ausgewählt ist aus Tetrahydrofuran, Dichlormethan, Chloroform und beliebigen Mischungen vorgenannter Lösemittel.

15. Verfahren nach Anspruch 14, **dadurch gekennzeichnet, dass** die Lösemittel getrocknet eingesetzt werden und der Schritt der Aktivierung der Hydroxylgruppen unter Schutzgasatmosphäre, insbesondere Stickstoffatmosphäre durchgeführt wird.

16. Verfahren nach Anspruch 15, **dadurch gekennzeichnet, dass** bei dem Schritt der Aktivierung der Hydroxylgruppen ein basisches Agens der Reaktionsmischung zugegeben wird, um freigesetzte Wasserstoffionen abzufangen.

17. Verfahren nach Anspruch 16, **dadurch gekennzeichnet, dass** das basische Agens ausgewählt ist aus Diethylamin, Triethylamin und Hünig-Base.

18. Verfahren nach einem der Ansprüche 9 bis 17, **dadurch gekennzeichnet, dass** die Polyesterkomponente mit den aktivierten Hydroxylgruppen in einer modifizierten Finckelstein-Reaktion umgesetzt wird, um die aktivierten Hydroxylgruppen in Halogenidgruppen zu überführen, bevor die Polyesterkomponente mit der Polypeptidkomponente umgesetzt wird.

19. Verfahren nach einem der Ansprüche 9 bis 18, **dadurch gekennzeichnet, dass** die Umsetzung der Polyester- mit der Polypeptidkomponente in einem polaren Lösemittel durchgeführt wird, welches ausgewählt ist aus DMF, DMSO und Lösemittelmischungen wie z.B. Ethylacetat/Wasser, Aceton/Wasser, THF/Wasser, CHCl_{3/}Wasser und CH₂Cl₂/Wasser.

20. Polyester-Polypeptid-Blockcopolymer, erhältlich nach einem Verfahren gemäß einem der voranstehenden Ansprüche.

## Claims

1. A depot medicament having a pharmacological active ingredient and a carrier, in particular for parenteral administration, **characterized in that** the carrier is produced with use of a carrier material which comprises a carrier polymer formed from a polypeptide and a biodegradable polyester covalently linked thereto.

2. A depot medicament according to Claim 1, **characterized in that** the ratio by weight of polyester content to polypeptide content is from 1:99 to 99:1.

3. A depot medicament according to Claim 1 or 2, **characterized in that** the ratio by weight of polyester content to polypeptide content is from 30:70 to 99:1.

4. A depot medicament according to any of Claims 1 to 3, **characterized in that** the polyester is selected from polyglycolides, poly(D,L-lactide-co- glycolides), polyalkylene glycol-polylactide block copolymers, polyalkylene glycol-PLGA block copolymers, POP-POE-PLA block copolymers, POP-PLA block copolymers, POP-PLGA block copolymers, POE-PLGA block copolymers, poly-ε-caprolactams, stereoisomeric lactide oligomers, oligolactides (n ≥ 3) or polylactides.

5. A depot medicament according to any of Claims 1 to 4, **characterized in that** the polypeptide is selected from collagen, gelatin, globular proteins or other hydrogel-forming proteins, enzymes and protein degradation products, which may be chemically and/or enzymatically modified.

6. A depot medicament according to any of Claims 1 to 5, **characterized in that** the linkage of polypeptide and polyester takes place via a free hydroxy function of the polyester with a reactive group of the polypeptide.

7. A depot medicament according to any of Claims 1 to 6, **characterized in that** the linkage of polypeptide and polyester takes place via a free carboxyl function of the polyester, or a carbonyl function obtained by oxidation of the hydroxyl function, with a reactive group of the polypeptide.

8. A depot medicament according to Claim 6 or 7, **characterized in that** the reactive group of the polypeptide is an amino function.

9. A method for the production of a carrier material for depot medicaments, **characterized by** the steps of
reaction of a polyester component with an activating agent in order to convert hydroxyl groups of the polyester component into an activated form,
addition of a polypeptide component and reaction of reactive groups of the polypeptide with the activated hydroxyl groups of the polyester to form covalent bonds.

10. A method according to Claim 9, **characterized in that** the hydroxyl groups are converted with the activating agent into good leaving groups.

11. A method according to Claim 9 or 10, **characterized in that** the polyester component is selected from polyglycolides, poly(D,L-lactide-co-glycolides), polyalkylene glycol-polylactide block copolymers, polyalkylene glycol-PLGA block copolymers, POP-POE-PLA block copolymers, POP-PLA block copolymers, POP-PLGA block copolymers, POE-PLGA block copolymers, poly-ε-caprolactams, stereoisomeric lactide oligomers, oligolactides (n ≥ 3) or polylactides.

12. A method according to any of Claims 9 to 11, **characterized in that** the polypeptide component is selected from collagen, gelatin, globular proteins or other hydrogel-forming proteins, enzymes and protein degradation products.

13. A method according to any of Claims 9 to 12, **characterized in that** the activating agent is selected from p-toluenesulfonyl chloride, methanesulfonyl chloride, p-bromobenzenesulfonyl chloride, p-nitrobenzenesulfonyl chloride and trifluoromethanesulfonyl chloride.

14. A method according to any of Claims 9 to 13, **characterized in that** the step of activation of the hydroxyl groups of the polyester component is carried out in a solvent which is selected from tetrahydrofuran, dichloromethane, chloroform and any mixtures of the aforementioned solvents.

15. A method according to Claim 14, **characterized in that** the solvents are employed dry, and the step of activation of the hydroxyl groups is carried out under a protective gas atmosphere, in particular nitrogen atmosphere.

16. A method according to Claim 15, **characterized in that** a basic agent is added to the reaction mixture in the step of activation of the hydroxyl groups in order to trap liberated hydrogen ions.

17. A method according to Claim 16, **characterized in that** the basic agent is selected from diethylamine, triethylamine and Hünig's base.

18. A method according to any of Claims 9 to 17, **characterized in that** the polyester component with the activated hydroxyl groups is reacted in a modified Finckelstein reaction in order to convert the activated hydroxyl groups into halide groups, before the polyester component is reacted with the polypeptide component.

19. A method according to any of Claims 9 to 18, **characterized in that** the reaction of the polyester component with the polypeptide component is carried out in a polar solvent which is selected from DMF, DMSO and solvent mixtures such as, for example, ethyl acetate/water, acetone/water, THF/water, CHCl₃/water and CH₂Cl₂/water.

20. A polyester-polypeptide block copolymer obtainable by a method according to any of the preceding claims.

## Revendications

1. Médicament retard comportant une matière active pharmacologique et un support, en particulier pour administration parentérale, **caractérisé en ce que** le support est produit en utilisant un matériau de support qui comprend un polymère de support formé à partir d'un polypeptide et d'un polyester biodégradable lié de manière covalente à ce dernier.

2. Médicament retard selon la revendication 1, **caractérisé en ce que** le rapport de poids entre la part de polyester et la part de polypeptide est de 1 :99 à 99 :1.

3. Médicament retard selon la revendication 1 ou 2, **caractérisé en ce que** le rapport de poids entre la part de polyester et la part de polypeptide est de 30 :70 à 99 :1.

4. Médicament retard selon une des revendications 1 à 3, **caractérisé en ce que** le polyester est sélectionné parmi les polyglycolides, les poly(D,L-lactide-coglycolides), les copolymères à blocs polyalkylène glycol-polylactide, les copolymères à blocs polyalkylène glycol-PLGA, les copolymères à blocs POP-POE-PLA, les copolymères à blocs POP-PLA, les copolymères à blocs POP-PLGA, les copolymères à blocs POE-PLGA, les poly-ε-caprolactames, les oligomères-lactides stéréoisomériques, les oligolactides (n ≥ 3) ou les polylactides.

5. Médicament retard selon une des revendications 1 à 4 **caractérisé en ce que** le polypeptide est sélectionné parmi le collagène, la gélatine, les protéines globulaires ou autres protéines formant des hydrogels, les enzymes et les produits de la dégradation des protéines qui peuvent être modifiés chimiquement et/ou enzymatiquement.

6. Médicament retard selon une des revendications 1 à 5, **caractérisé en ce que** la liaison du polypeptide et du polyester est effectuée par l'intermédiaire d'une fonction hydroxyle libre du polyester avec un groupe réactif du polypeptide.

7. Médicament retard selon une des revendications 1 à 6, **caractérisé en ce que** la liaison du polypeptide et du polyester est effectuée par l'intermédiaire d'une fonction carboxyle libre du polyester, ou une fonction carbonyle obtenue par oxydation de la fonction hydroxyle avec un groupe réactif du polypeptide.

8. Médicament retard selon la revendication 6 ou 7, **caractérisé en ce que** le groupe réactif du polypeptide est une fonction amino.

9. Procédé de production d'un matériau de support pour des médicaments retard, **caractérisé par** les étapes suivantes consistant à :
transformer un composant polyester avec un agent activant, pour convertir les groupes hydroxyles du composant polyester sous une forme activée,
ajouter un composant polypeptide, et faire réagir les groupes réactifs du polypeptide avec les groupes hydroxyles activés du polyester pour former des liaisons covalentes.

10. Procédé selon la revendication 9, **caractérisé en ce que** les groupes hydroxyles sont transformés par l'agent activant en bons groupes partants.

11. Procédé selon la revendication 9 ou 10, **caractérisé en ce que** le composant polyester est sélectionné parmi les polyglycolides, les poly(D,L-lactide-co-glycolides), les copolymères à blocs polyalkylène glycol-polylactide, les copolymères à blocs polyalkylène glycol-PLGA, les copolymères à blocs POP-POE-PLA, les copolymères à blocs POP-PLA, les copolymères à blocs POP-PLGA, les copolymères à blocs POE-PLGA, les poly-ε-caprolactames, les oligomères-lactides stéréoisomériques, les oligolactides (n ≥ 3) ou les polylactides.

12. Procédé selon une des revendications 9 à 11, **caractérisé en ce que** le composant polypeptide est sélectionné parmi le collagène, la gélatine, les protéines globulaires ou autres protéines formant des hydrogels, les enzymes et les produits de la dégradation des protéines.

13. Procédé selon une des revendications 9 à 12 **caractérisé en ce que** l'agent activant est sélectionné parmi le chlorure de p-toluène sulfonyle, le chlorure de méthane sulfonyle, le chlorure de p-bromobenzène sulfonyle, le chlorure de p-nitrobenzène sulfonyle, et le chlorure de trifluorométhane sulfonyle.

14. Procédé selon une des revendications 9 à 13, **caractérisé en ce que** l'étape de l'activation des groupes hydroxyles des composants polyester est effectuée dans un solvant qui est sélectionné parmi le tétrahydrofurane, le dichlorométhane, le chloroforme, et n'importe quel mélange des solvants cités ci-dessus.

15. Procédé selon la revendication 14, **caractérisé en ce que** le solvant est utilisé sec, et **en ce que** l'étape de l'activation des groupes hydroxyles est effectuée sous atmosphère inerte, en particulier sous atmosphère azotée.

16. Procédé selon la revendication 15, **caractérisé ce que** lors de l'étape de l'activation des groupes hydroxyles, on ajoute au mélange réactif un agent basique pour récupérer les ions d'azote libérés.

17. Procédé selon la revendication 16, **caractérisé en ce que** l'agent basique est sélectionné parmi la diéthylamine, la triéthylamine, et la base de Hünig.

18. Procédé selon une des revendications 9 à 17, **caractérisé en ce que** le composant polyester est transformé par l'agent activant avec les groupes hydroxyles dans une réaction de Finckelstein modifiée, pour convertir les groupes hydroxyles en groupes halogènes avant que le composant polyester ne soit transformé avec le composant polypeptide.

19. Procédé selon une des revendications 9 à 18, **caractérisé en ce que** la transformation du composant polyester avec le composant polypeptide est effectuée dans un solvant polaire qui est sectionné parmi le DMF, le DMSO, et des mélanges de solvants tels qu'acétate d'éthyle/eau, acétone/eau, THF/eau, CHCl₃/eau et CH₂Cl₂/eau.

20. Copolymère à blocs polyester-polypeptide, obtenu selon un procédé selon une des revendications précédentes.
